# EUROPÄISCHE PATENTANMELDUNG

(11) **EP 4 643 769 A1**
(43) Veröffentlichungstag der Anmeldung: **05.11.2025**
(21) Anmeldenummer: 24174237.8
(22) Anmeldetag: 04.05.2024
(51) Int. Cl.: A61B 5/08, G01N 33/497

(54) **NICHT-INVASIVES VERFAHREN ZUR DIAGNOSE VON DARMERKRANKUNGEN**

(71) Anmelder: VOC - Advanced Breath Diagnostics GmbH, 91052 Erlangen (DE)
(72) Erfinder: VOIGT, Wieland, 91096 Möhrendorf (DE); MEKONNEN, Tessema Fenta, 91301 Forchheim (DE); SINGH, Stuti, 91052 Erlangen (DE)
(74) Vertreter: Simandi, Claus

(57) **Zusammenfassung**

Die vorliegende Erfindung betrifft ein nicht-invasives Verfahren zur Diagnose, Prädiktion, Risikostratifizierung und Therapiesteuerung von Darmerkrankungen an Patienten, wobei durch Darmbakterien gebildete Gase in dem Ausatemgas bestimmt werden, insbesondere ein Vortest und die Stratifizierung eines Patienten.

## Beschreibung

Die vorliegende Erfindung betrifft ein nicht-invasives Verfahren zur Diagnose, Prädiktion, Risikostratifizierung und Therapiesteuerung von Darmerkrankungen an Patienten, wobei durch Darmbakterien gebildete Gase in dem Ausatemgas bestimmt werden, insbesondere ein Vortest und die Stratifizierung eines Patienten.

SIBO (Small Intestinal Bacterial Overgrowth) ist eine Fehlbesiedlung des Dünndarms von Bakterien, die sich mit unterschiedlichen Symptomen wie Blähungen, Bauchschmerzen, Obstipation und chronischer Diarrhoe (Übersicht in (1)) manifestieren kann. Die Prävalenz ist hoch und liegt je nach Studie zwischen 2,5% und 22% der Bevölkerung (2-4). Die Symptomatik von SIBO und dem noch häufiger auftretenden Reizdarmsyndrom ist häufig überlappend (5), so dass Teste für SIBO wichtig für eine gute Differentialdiagnostik und Therapie sind, umgekehrt häufig Fehldiagnosen existieren, die einer Behandlung entgegenstehen. Daher ist der Einsatz von Testen auf SIBO ebenfalls bei Verdacht auf das Reizdarmsyndrom unerlässlich. Für die Behandlung von SIBO stehen verschiedene Optionen zur Verfügung (6), in erster Linie die Verabreichung von Antibiotika/Phytobiotika neben diätischen Maßnahmen und Aufnahme von Probiotika.

Die genaue Ursache von SIBO ist bis heute unklar, jedoch können Faktoren wie Darmmotilitätsstörungen, anatomische Anomalien im Darm, bestimmte Erkrankungen wie Morbus Crohn oder Diabetes, sowie vorangegangene Darmoperationen u.a. das Risiko erhöhen.

Im Stand der Technik erfolgt die Diagnose von SIBO in einer Kombination aus Klinik und Atemteste, bei denen gemessen wird, ob bestimmte Gase wie Wasserstoff oder Methan in erhöhten Mengen im Atem vorhanden sind, nachdem eine Kohlenhydrat-Lösung eingenommen wurde (1, 7). Diese Gase werden als Stoffwechselprodukte von SIBO-verursachenden Bakterien nach Zugabe von Zuckern wie Glukose, Laktose oder Laktulose provoziert (7) und erlauben daher den spezifischen indirekten Nachweis der Dünndarmfehlbesiedlung. Manche Teste beruhen ausschließlich auf der Messung von H₂, was allerdings mit einer schlechteren Sensitivität und Spezifität gegenüber einem Test, der beide Gase bestimmt, einhergeht (8). Zudem wird die simultane Bestimmung von CO₂ in den Atemproben empfohlen, um die nicht-alveoläre Verdünnung der Atemprobe während der Probennahme abschätzen zu können (8,9). Der etablierte SIBO-Test erfordert eine umfangreiche Vorbereitung des Probanden, die besondere diätische Maßnahmen ggfs. eine eventuelle Anpassung der Medikation umfasst. Nach der Vorbereitung nimmt der Proband üblicherweise eine definierte Menge eines Zuckers (supra) auf und spendet im zeitlichen Abstand mehrere Atemproben, die anschließend auf die SIBO-typischen Ausatemgase untersucht werden. Die Messergebnisse werden im zeitlichen Verlauf betrachtet. Nach Richtlinien aus den USA und Europa ist ein SIBO-Atemtest bei einem Anstieg um 20 ppm H₂ von der Basislinie (8,10) bzw. ab einer absoluten Konzentration von 10ppm CH₄ (8) nach Zuckergabe als positiv zu bewerten.

Die Bestimmung von H₂, CH₄ und CO₂ aus Ausatemproben erfolgt in der klinischen Praxis entweder über Gaschromatographie oder Gassensoren. Entsprechende Teste sind kommerziell verfügbar und in der Literatur beschrieben (13-18).

Der Durchführung der etablierten Teste zum Nachweis von SIBO geht eine umfangreiche Vorbereitung voraus. So sollen beispielsweise schon Wochen vor dem Test keine Antibiotika oder auch Probiotika eingenommen werden, Tage vor dem Test soll auf ballaststoffreiche Lebensmittel wie Gemüse, insbesondere Bohnen und Cerealien sowie bestimmte Medikamente verzichtet werden, und am Tag des Testes soll die Mundhöhle mit Chlorhexidin desinfiziert werden (8, 10, 19).

Die aufwändige Vorbereitung mindert zum einen die Akzeptanz des Testes, zum anderen wirken sich Fehler bei der Vorbereitung auf die Aussagekraft des Testes aus. Für das Screening von Individuen auf Darmerkrankungen, insbesondere SIBO oder das Reizdarmsyndrom ist daher ein einfacher und leicht durchzuführender Vortest vor dem eigentlichen SIBO Atemtest zu fordern, um ausschließlich die Individuen der aufwändigen Vorbereitung zu unterziehen, bei denen eine gewisse Wahrscheinlichkeit für das Vorliegen von Darmerkrankungen, insbesondere SIBO oder dem Reizdarmsyndrom gegeben ist.

Der Atemtest ist eine nicht-invasive Methode zur Diagnose von "Small Intestinal Bacterial Overgrowth" (kurz: SIBO) und verläuft wie folgt.

Vorbereitung: In den Tagen vor der Durchführung des SIBO-Tests wird der Proband gebeten, bestimmte Nahrungsmittel und Medikamente im Sinne einer Diät zu vermeiden, die das Testergebnis beeinflussen können. Dazu gehören oft Antibiotika, Protonenpumpenhemmer und bestimmte ballaststoffreiche Lebensmittel.

Testsubstanz: Der Proband erhält eine Testsubstanz, die normalerweise Laktulose oder Glukose ist. Diese Substanzen sind vorzugsweise Kohlenhydrate, die normalerweise im Dünndarm nicht verdaut werden, aber von Darmbakterien fermentiert werden können, und eine Gasentwicklung provozieren.

Atemprobenentnahme: Der Proband atmet in spezielle Atembeutel oder Röhrchen, die Atemgase absorbieren können. Die erste Atemprobe wird vor der Einnahme der Testsubstanz genommen, um den Grundlinienwert (Basiswert) zu bestimmen. Anschließend nimmt der Proband die Testsubstanz auf.

Messungen/Bestimmungen: Nachdem die Testsubstanz eingenommen wurde, werden in regelmäßigen Abständen Atemproben entnommen, normalerweise alle 15-30 Minuten über einen Zeitraum von einigen Stunden. Diese Proben werden dann auf ihren Gehalt an Wasserstoff (H₂) und/oder Methan (CH₄) untersucht, die von den Darmbakterien produziert werden, wenn sie die Testsubstanz fermentieren.

Interpretation der Ergebnisse: Ein Anstieg des Wasserstoff- und / oder Methangehalts im Atem deutet auf eine bakterielle Überwucherung im Dünndarm hin. Die Muster der Wasserstoff- und Methanproduktion können auch helfen, zwischen verschiedenen Arten von SIBO zu unterscheiden.

Überraschenderweise wurde nunmehr gefunden, dass ein Stufentest zur Bestimmung der Gehalte an den Gasen H₂ und CH₄ im Ausatmen eines Probanden zu zwei Zeitpunkten und im zeitlichen Abstand nach einem Kurzzeitfasten, nämlich eine Basisprobe gefolgt von einer Mahlzeit und anschließender zweiter Probe, den Erfolg für einen nachfolgenden SIBO-Test erleichtert bzw. die diagnostische Wertigkeit eines SIBO-Tests erhöht.

Daher ist eine Aufgabe der vorliegenden Erfindung, die Bereitstellung eines Vortests, sodass Patienten für eine weitere Diagnose oder Prädiktion auf Darmerkrankungen stratifiziert werden können.

Daher betrifft die Erfindung ein nicht-invasives Verfahren zur Diagnose oder Prädiktion von Darmerkrankungen, wobei durch Darmbakterien gebildete Gase in dem Ausatemgas eines Probanden bestimmt werden, mit den folgenden Schritten:
a.) Durchführen eines Kurzzeitfastens von mindestens 6 Stunden und / oder maximal 12 Stunden eines Probanden,
b.) Bestimmung der ppm Gehalte von H₂ und CH₄ in dem Ausatemgas eines Probanden aus a.),
c.) Aufnahme einer Mahlzeit eines Probanden aus b.),
d.) Bestimmung der ppm Gehalte von H₂ und CH₄ in dem Ausatemgas eines Probanden aus c.),

d.1) wobei eine Zunahme der Gehalte von mindestens mehr als 10 ppm von H₂ und von mindestens mehr als 10 ppm von CH₄ gegenüber
b.) indikativ für eine Darmerkrankung ist, und der Proband stratifiziert wird,
e.) die Diagnose oder Prädiktion von Darmerkrankungen durch ein zweites nicht-invasives Verfahren zur Diagnose oder Prädiktion von Darmerkrankungen zu verifizieren, wobei H₂ und/oder CH₄ in dem Ausatemgas eines Probanden bestimmt werden,
   oder
d.2) wobei eine Zunahme der Gehalte von gleich oder weniger als 10 ppm von H₂ und von gleich oder weniger als 10 ppm von CH₄ gegenüber b.) nicht indikativ für SIBO ist, und der Proband stratifiziert wird,
f.) die Diagnose oder Prädiktion von Darmerkrankungen nicht durch ein zweites nicht-invasives Verfahren zur Diagnose oder Prädiktion von Darmerkrankungen, insbesondere SIBO, zu verifizieren, wobei H₂ und/oder CH₄ in dem Ausatemgas eines Probanden bestimmt werden.

Weiterhin betrifft die Erfindung in einer weiteren bevorzugten Ausführungsform ein nicht-invasives Verfahren zur Diagnose oder Prädiktion von Darmerkrankungen, wie vorstehend, wobei der Schritt e.), folgende weitere Schritte aufweist:
i.) Durchführen einer Diät von mindestens 3 Tagen eines Probanden,
ii.) Bestimmung der ppm Gehalte von H₂ und CH₄ in dem Ausatemgas eines Probanden aus i.),
iii.) Aufnahme von Zucker, insbesondere Lactulose oder Glucose, eines Probanden aus ii.),
iv.) Bestimmung der ppm Gehalte von H₂ und/oder CH₄ in dem Ausatemgas eines Probanden aus iii.).

Weiterhin betrifft die Erfindung in einer weiteren bevorzugten Ausführungsform ein nicht-invasives Verfahren zur Diagnose oder Prädiktion von Darmerkrankungen, wie vorstehend, wobei der Schritt e.), folgenden weiteren Schritt aufweist:
v.) wobei jeweils eine Zunahme der Gehalte von mindestens mehr als 10 ppm von H₂ und/oder von mindestens mehr als 10 ppm von CH₄ gegenüber ii.) indikativ für SIBO ist.

Ferner betrifft die Erfindung in einer weiteren bevorzugten Ausführungsform ein nicht-invasives Verfahren zur Diagnose oder Prädiktion von Darmerkrankungen, wie vorstehend, wobei die zusätzliche Alternative d.3) zu d.1) oder d.2) folgt:
oder
d.3) wobei eine Zunahme der Gehalte von gleich oder weniger als 10 ppm von H₂ und von gleich oder weniger als 10 ppm CH₄ gegenüber b.) nicht indikativ für SIBO ist, und der Proband stratifiziert wird,
f.) die Diagnose oder Prädiktion von Darmerkrankungen durch ein weiteres Verfahren zur Diagnose oder Prädiktion von mindestens einer Darmerkrankung ausgewählt aus der Gruppe Reizdarmsyndrom (IBS),Laktose- oder Fruktosemalabsorption, chronisch entzündliche Darmerkrankungen (CED), insbesondere Morbus Crohn und Colitis ulcerosa, Zöliakie, funktionelle Dyspepsie durchzuführen.

Die Darmerkrankungen "Reizdarmsyndrom (IBS), Laktose- oder Fruktosemalabsorption, chronisch entzündliche Darmerkrankungen (CED), insbesondere Morbus Crohn und Colitis ulcerosa, Zöliakie, funktionelle Dyspepsie" weisen ähnliche Symptome wie SIBO auf. Diese Darmerkrankungen sind z.B. im Pschyrembel beschrieben oder der WHO (ICD) bekannt, und geeignete Verfahren zur Diagnose oder Prädiktion sind dem Fachmann bekannt.

Im Sinne dieser Erfindung sind Darmerkrankungen, solche wie SIBO, wobei SIBO besonders bevorzugt ist, jedoch auch solche Darmerkrankungen, die in der Symptomatik zu SIBO ähnlich sind, nämlich Reizdarmsyndrom (IBS), Laktose- oder Fruktosemalabsorption, chronisch entzündliche Darmerkrankungen (CED), insbesondere Morbus Crohn und Colitis ulcerosa, Zöliakie oder funktionelle Dyspepsie. Insbesondere sind solche Darmerkrankungen erfindungsgemäß umfasst, die eine Malabsorption aufweisen.

Weiterhin betrifft die Erfindung in einer weiteren bevorzugten Ausführungsform ein nicht-invasives Verfahren zur Diagnose oder Prädiktion von Darmerkrankungen, wie vorstehend, wobei eine Zunahme der ppm Gehalte von H₂ und/oder CH₄ innerhalb von 60 bis 100 Minuten erfolgt, insbesondere 80 Minuten.

Eine weitere Aufgabe der Erfindung ist ebenfalls die Therapiesteuerung mittels einer Prädiktion oder Diagnose von Darmerkrankungen, vorzugsweise SIBO, wobei ein Proband nach einem erfindungsgemäßen Verfahren untersucht wird, mit der Maßgabe, dass der Proband Medikamente, insbesondere Antibiotika/Phytobiotika zur Behandlung einer Darmerkrankung erhält oder eine Diät zur Behandlung einer Darmerkrankung einhält, insbesondere zur Behandlung einer Malabsorption, sodass ein Therapieerfolg oder Therapieverbesserung erfolgen kann.

Der Begriff "Fasten(periode)" im Sinne dieser Erfindung ist ein geplanter Zeitraum, bei der Menschen sich bewusst der völligen Enthaltung von Nahrung und Getränken andere als kleine Mengen Wasser unterziehen.

Der Begriff "Mahlzeit" im Sinne dieser Erfindung ist eine geplante Gelegenheit, bei der Menschen Nahrung zu sich nehmen, um ihren Hunger zu stillen und ihren Körper mit Nährstoffen zu versorgen. Eine Mahlzeit kann insbesondere Kohlenhydrate enthalten.

Der Begriff "Kurzzeitfastens von mindestens 6 Stunden und / oder maximal 12 Stunden" im Sinne dieser Erfindung bedeutet, dass ein bestimmtes Zeitfenster für das Essen und Fasten festgelegt wird. Innerhalb dieser Fastenzeit erfolgt keine Nahrungsaufnahme, insbesondere mittels einer Mahlzeit.

"Diagnose" oder "Prädiktion" im Sinne dieser Erfindung bedeutet die positive Feststellung oder Vorhersage / Prognose bzw. Wahrscheinlichkeit des Ein- und Auftretens einer Darmerkrankung, insbesondere SIBO.

Der Begriff der Diagnose umfasst weiterhin die medizinische Diagnostik und diesbezügliche Untersuchungen, insbesondere die in-vitro Diagnostik und Labordiagnostik.

Ferner betrifft die Erfindung ein Verfahren zum Stratifizieren, insbesondere zur Risikostratifizierung und / oder Therapiesteuerung eines Patienten.

"Stratifizieren oder Therapiesteuerung" im Sinne dieser Erfindung bedeutet, dass das erfindungsgemäße Verfahren Entscheidungen zur Behandlung und Therapie des Probanden erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie des Therapieverlaufs bzw. Ätiologie oder Klassifizierung von Darmerkrankungen, insbesondere SIBO. Weiterhin bedeutet "stratifizieren" im Sinne dieser Erfindung, die anspruchsgemäße Weisung, dass der Proband ein weiteres Verfahren zur Diagnose und Prädiktion von Darmerkrankungen erhält oder eben nicht erhält (supra).

In einer weiteren Ausführungsform der Erfindung umfasst der Begriff "Stratifizierung" insbesondere die Risikostratifizierung mit der Prädiktion eines "outcome" eines nachteiligen gesundheitlichen Ereignisses, insbesondere, dass eine Darmerkrankung vorliegt.

Im Rahmen dieser Erfindung wird unter "Proband" ein beliebiger Mensch oder Säugetier verstanden, insbesondere ein Patient. Besonders bevorzugt sind Patienten, die bereits Symptome aufweisen, und zwar Blähungen, Bauchschmerzen, Durchfall, Verstopfung, Nährstoffmangel und Gewichtsverlust aufgrund Malabsorption.

Die Erfindung wird nun in den folgenden Beispielen näher beschrieben, ohne darauf beschränkt zu sein.

### Beispiele:

### Beispiel 1:

Für den Spot-Test werden Proben mit Plastikstrohhalmen von Medi-Inn (Hirten, Deutschland), Artikelnummer: 93652-Karton, EAN: 4260655961231 und Glasröhrchen von Zhejiang ALWSCI Technologies Co., Ltd.) verwendet.

Die Messungen erfolgen mittels Gaschromatographie mit Barriereentladungsdetektor (GC-BID), ausgestattet mit einer Carboxen PLOT-Säule (30m × 0,53mm und 0,50 µm Schichtdicke) und einem AOC-6000 Plus Autosampler (Shimadzu, Duisburg, Deutschland). Der Temperaturgradient beträgt 35°C (Haltedauer 3 min) und wird rampenförmig auf 80°C (50 °C/min, Haltedauer 5 min) und weiter auf 170 °C (50 °C/min, Haltedauer 2 min) erhöht. Die BID und die Injektionstemperatur werden auf 250 °C gehalten. Das Injektionsvolumen und der Trägergasfluss von Helium (Reinheit 99,9999 %) betragen 100 µL bzw. 8 mL/min.

Zur Probennahme atmet der Proband durch den Strohhalm aus; beim letzten Drittel eines Ausatemzyklus wird das untere Ende des Strohhalms in ein Glasröhrchen gesteckt und dort in Bodennähe positioniert, wobei weiterhin ausgeatmet wird. Sodann wird der Strohhalm - bei konstantem Ausatmen - langsam in Richtung Öffnung des Glasröhrchens bewegt. Ist der Strohhalm beim Ende des Ausatemzyklus am oberen Rand des Glasröhrchens angekommen, wird dieses mit einem Drehverschluss verschlossen. Für Vergleichsmessungen bzw. den Haupttest erfolgt die Probennahme mit dem EasySampler^{®} Kit der Firma QuinTron (3712 West Pierce Street, Milwaukee, WI, USA) nach Angaben des Herstellers. Die Vorbereitung für den Haupt- bzw. Vergleichstest erfolgt im Einklang mit den Richtlinien für SIBO-Diagnostik bzw. wie in der Packungsbeilage des VOC-SIBO Testes der Firma VOC-Advanced Breath Diagnostics GmbH, Henkestraße 91, 91052 Erlangen, Deutschland) beschrieben.

Anschließend werden die Proben (Vortest und Haupt- bzw. Vergleichstest) mit einem Gaschromatographie-System der Firma Shimadzu, Modell GC-2030 mit dem Autosampler AOC-6000 Plus und einer SH-Rt-Molsieve 5A P/N 221-75763-30 Säule analysiert. Die Analyse der Messdaten erfolgte an einer Kalibrationskurve, die mittels Gasproben bekannter Konzentration und metrologischer Rückführbarkeit auf internationale Standards erzeugt worden ist (Gase sind von Firma All-In Gas, Emil-Riedel-Straße 1, 80538 München, Deutschland bezogen).

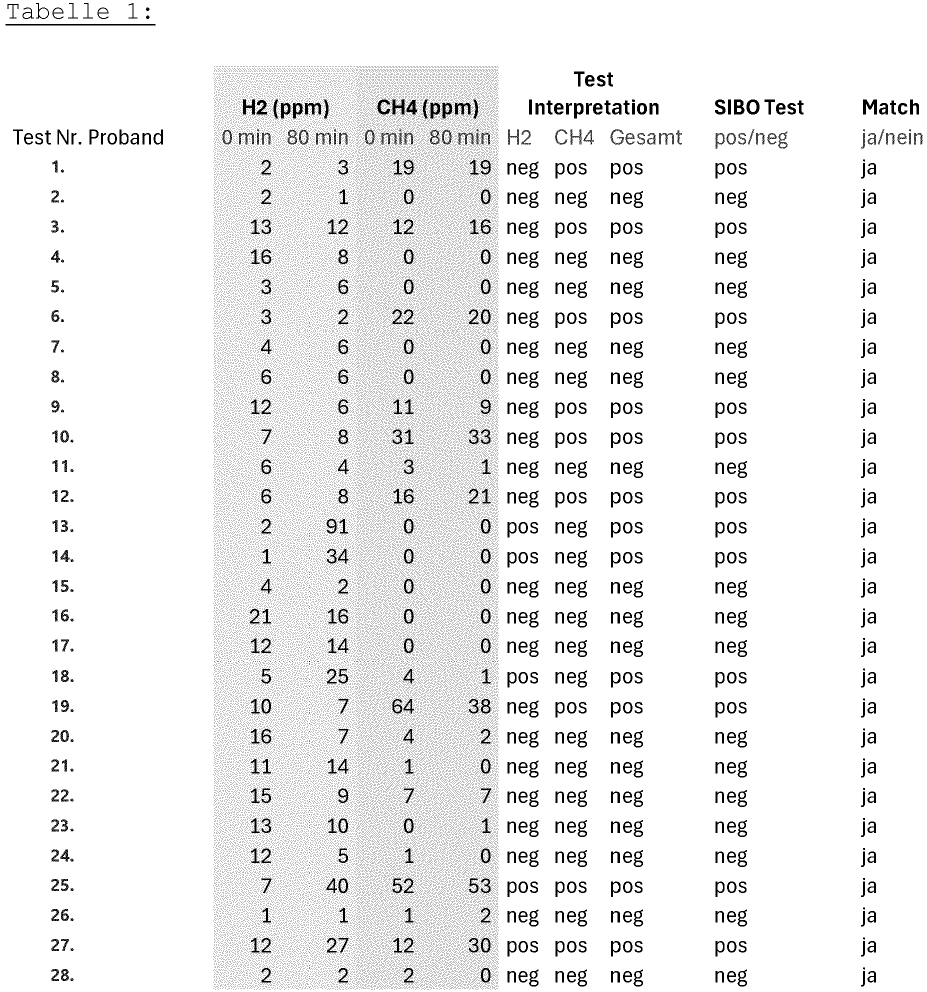

Interpretation: SPOT positiv bei Anstieg H₂ von Zeitpunkt 0 min auf 80 min um ≥ 10 ppm und CH₄ ≥ 10 ppm zu beliebigen Zeitpunkt. Haupttest positiv bei Anstieg von H₂ innerhalb von 90 min nach Substratgabe ≥ 20 ppm und CH₄ ≥ 10 ppm zu beliebigen Zeitpunkt.

Schlussfolgerung: Spot Test ist geeignet zur Vorselektion von Patienten für die Anwendung des Volltests auf SIBO, aber auch Laktose oder Fruktose Malabsorption.

Ergebnis: Vorhersage des Spot-Test bei Cut off (Schwellenwert) von mehr als 10 ppm für Wasserstoff und Methan zu 100% korrekt bei N=28.

Literatur:
1. Skrzyd 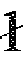 o-Radomańska B, Cukrowska B. How to Recognize and Treat Small Intestinal Bacterial Overgrowth? J Clin Med. 2022 Oct 12;11(20):6017)
2. Krajicek, E.J.; Hansel, S.L. Small Intestinal Bacterial Overgrowth: A Primary Care Review. Mayo Clin. Proc. 2016, 91, 1828-1833
3. Achufusi, T.G.O.; Sharma, A.; Zamora, E.A.; Manocha, D. Small Intestinal Bacterial Overgrowth: Comprehensive Review of Diagnosis, Prevention, and Treatment Methods. Cureus 2020, 12, e8860
4. Grace, E.; Shaw, C.; Whelan, K.; Andreyev, H.J.N. Review article: Small intestinal bacterial overgrowth-Prevalence, clinical features, current and developing diagnostic tests, and treatment. Aliment. Pharmacol. Ther. 2013, 38, 674-688
5. Ghoshal UC, Shukla R, Ghoshal U. Small Intestinal Bacterial Overgrowth and Irritable Bowel Syndrome: A Bridge between Functional Organic Dichotomy. Gut Liver. 2017 Mar 15;11(2):196-208. doi: 10.5009/gnl16126. PMID: 28274108; PMCID: PMC5347643.
6. Rao S.S.C., Bhagatwala J. Small Intestinal Bacterial Overgrowth: Clinical features and therapeutic management: Clinical features and therapeutic management. Clin. Transl. Gastroenterol. 2019;10:e00078.
7. Losurdo G., Leandro G., Ierardi E., Perri F., Barone M., Principi M., Di Leo A. Breath Tests for the Non-invasive Diagnosis of Small Intestinal Bacterial Overgrowth: A Systematic Review with Meta-analysis. J. Neurogastroenterol.
8. Rezaie A, Buresi M, Lembo A, Lin H, McCallum R, Rao S, Schmulson M, Valdovinos M, Zakko S, Pimentel M. Hydrogen and Methane-Based Breath Testing in Gastrointestinal Disorders: The North American Consensus. Am J Gastroenterol. 2017 May;112(5):775-784.
9. Goldoni M, Corradi M, Mozzoni P, Folesani G, Alinovi R, Pinelli S, Andreoli R, Pigini D, Tillo R, Filetti A, Garavelli C, Mutti A. Concentration of exhaled breath condensate biomarkers after fractionated collection based on exhaled CO2 signal. J Breath Res. 2013 Mar;7(1)
10. Gasbarrini A, Corazza GR, Gasbarrini G, Montalto M, Di Stefano M, Basilisco G, Parodi A, Usai-Satta P, Vernia P, Anania C, Astegiano M, Barbara G, Benini L, Bonazzi P, Capurso G, Certo M, Colecchia A, Cuoco L, Di Sario A, Festi D, Lauritano C, Miceli E, Nardone G, Perri F, Portincasa P, Risicato R, Sorge M, Tursi A; 1st Rome H2-Breath Testing Consensus Conference Working Group. Methodology and indications of H2-breath testing in gastrointestinal diseases: the Rome Consensus Conference. Aliment Pharmacol Ther. 2009 Mar 30;29 Suppl 1:1-49.
11. Banik GD, De A, Som S, Jana S, Daschakraborty SB, Chaudhuri S, Pradhan M. Hydrogen sulphide in exhaled breath: a potential biomarker for small intestinal bacterial overgrowth in IBS. J Breath Res. 2016 May 10;10(2):026010. doi: 10.1088/1752-7155/10/2/026010. PMID: 27163246.
12. Takakura W, Pimentel M. Small Intestinal Bacterial Overgrowth and Irritable Bowel Syndrome - An Update. Front Psychiatry. 2020 Jul 10;11:664. doi: 10.3389/fpsyt.2020.00664. PMID: 32754068; PMCID: PMC7366247.
13. Shrestha A, Prodhan UK, Mitchell SM, Sharma P, Barnett MPG, Milan AM, Cameron-Smith D. Validity of a Portable Breath Analyser (AIRE) for the Assessment of Lactose Malabsorption. Nutrients. 2019 Jul 17;11(7):1636
14. Guillermo Barahona, Barry Mc Bride, Áine Moran, Sahar Hawamdeh, Luisa Villatoro, Robert Bums, Bo Konings, Robert Bulat, Megan McKnight, Claire Shortt, Pankaj J. Pasricha;Improving the Diagnosis of SIBO Using an At-Home Handheld App Connected Breath Analysis Device (AIRE). medRxiv preprint doi: https://doi.org/10.1101/2022.04.21.22274143; this version posted April 21, 2022.
15. Saad RJ, Chey WD. Breath testing for small intestinal bacterial overgrowth: maximizing test accuracy. Clin Gastroenterol Hepatol. 2014 Dec;12(12):1964-72;
16. Dharmawardana N, Goddard T, Woods C, Watson DI, Butler R, Ooi EH, Yazbeck R. Breath methane to hydrogen ratio as a surrogate marker of intestinal dysbiosis in head and neck cancer. Sci Rep. 2020 Sep 14;10(1):15010
17. de Lacy Costello BP, Ledochowski M, Ratcliffe NM. The importance of methane breath testing: a review. J Breath Res. 2013 Jun;7(2):024001.
18. Gao, Fan & Wang, Min & Zhang, Xusheng & Zhang, Junyu & Xue, Yingying & Wan, Hao & Wang, Ping. (2018). Simultaneous Detection of Hydrogen and Methane in Breath for the Diagnosis of Small Intestinal Bacterial Overgrowth by Fast Gas Chromatography. Analytical Methods. 10. 10.1039/C8AY01451E.
19. Piqué JM, Pallarés M, Cusó E, Vilar-Bonet J, Gassull MA. Methane production and colon cancer. Gastroenterology. 1984 Sep;87(3):601-5. PMID: 6745612.
20. Li L, Zhang XY, Yu JS, Zhou HM, Qin Y, Xie WR, Ding WJ, He XX. Ability of lactulose breath test results to accurately identify colorectal polyps through the measurement of small intestine bacterial overgrowth. World J Gastrointest Surg. 2023 Jun 27;15(6):1138-1148. doi: 10.4240/wjgs.v15.i6.1138. PMID: 37405104; PMCID: PMC10315122.
21. Lin H, Yu Y, Zhu L, Lai N, Zhang L, Guo Y, Lin X, Yang D, Ren N, Zhu Z, Dong Q. Implications of hydrogen sulfide in colorectal cancer: Mechanistic insights and diagnostic and therapeutic strategies. Redox Biol. 2023 Feb;59:102601. doi: 10.1016/j.redox.2023.102601. Epub 2023 Jan 7. PMID: 36630819; PMCID: PMC9841368.
22. Saad RJ, Chey WD. Breath testing for small intestinal bacterial overgrowth: maximizing test accuracy. Clin Gastroenterol Hepatol. 2014 Dec;12(12):1964-72; quiz e119-20.
23. Gottlieb K, Le C, Wacher V, Sliman J, Cruz C, Porter T, Carter S. Selection of a cutoff for high- and low-methane producers using a spot-methane breath test: results from a large north American dataset of hydrogen, methane and carbon dioxide measurements in breath. Gastroenterol Rep (Oxf). 2017 Aug;5(3):193-199. doi: 10.1093/gastro/gow048. Epub 2017 Jan 27. Erratum in: Gastroenterol Rep (Oxf). 2018 Aug;6(3):242. PMID: 28130375; PMCID: PMC5554383.

## Patentansprüche

1. Nicht-invasives Verfahren zur Diagnose oder Prädiktion von Darmerkrankungen, wobei durch Darmbakterien gebildete Gase in dem Ausatemgas eines Probanden bestimmt werden, mit den folgenden Schritten:
a.) Durchführen eines Kurzzeitfastens von mindestens 6 Stunden oder maximal 12 Stunden eines Probanden,
b.) Bestimmung der ppm Gehalte von H₂ und CH₄ in dem Ausatemgas eines Probanden aus a.),
c.) Aufnahme einer Mahlzeit eines Probanden aus b.),
d.) Bestimmung der ppm Gehalte von H₂ und CH₄ in dem Ausatemgas eines Probanden aus c.),
d.1) wobei eine Zunahme der Gehalte von mindestens mehr als 10 ppm von H₂ und von mindestens mehr als 10 ppm von CH₄ gegenüber b.) indikativ für eine Darmerkrankung ist, und der Proband stratifiziert wird,
e.) die Diagnose oder Prädiktion von Darmerkrankungen durch ein zweites nicht-invasives Verfahren zur Diagnose oder Prädiktion von Darmerkrankungen zu verifizieren, wobei H₂ und/oder CH₄ in dem Ausatemgas eines Probanden bestimmt werden,
oder
d.2) wobei eine Zunahme der Gehalte von gleich oder weniger als 10 ppm von H₂ und von gleich oder weniger als 10 ppm von CH₄ gegenüber b.) nicht indikativ für SIBO ist, und der Proband stratifiziert wird,
f.) die Diagnose oder Prädiktion von Darmerkrankungen nicht durch ein zweites nicht-invasives Verfahren zur Diagnose oder Prädiktion von Darmerkrankungen, insbesondere SIBO, zu verifizieren, wobei H₂ und/oder CH₄ in dem Ausatemgas eines Probanden bestimmt werden.

2. Nicht-invasives Verfahren zur Diagnose oder Prädiktion von Darmerkrankungen nach Anspruch 1, wobei der Schritt e.), folgende weitere Schritte aufweist:
i.) Durchführen einer Diät von mindestens 3 Tagen eines Probanden,
ii.) Bestimmung der ppm Gehalte von H₂ und CH₄ in dem Ausatemgas eines Probanden aus i.),
iii.) Aufnahme von Zucker, insbesondere Lactulose oder Glucose, eines Probanden aus ii.),
iv.) Bestimmung der ppm Gehalte von H₂ und/oder CH₄ in dem Ausatemgas eines Probanden aus iii.).

3. Nicht-invasives Verfahren zur Diagnose oder Prädiktion von Darmerkrankungen nach Anspruch 2, wobei der Schritt e.), folgenden weiteren Schritt aufweist:
v.) wobei jeweils eine Zunahme der Gehalte von mindestens mehr als 10 ppm von H₂ und/oder von mindestens mehr als 10 ppm von CH₄ gegenüber ii.) indikativ für SIBO ist.

4. Nicht-invasives Verfahren zur Diagnose oder Prädiktion von Darmerkrankungen nach Anspruch 1, wobei die zusätzliche Alternative d.3) folgt:
oder
d.3) wobei eine Zunahme der Gehalte von gleich oder weniger als 10 ppm von H₂ und von gleich oder weniger als 10 ppm CH₄ gegenüber b.) nicht indikativ für SIBO ist, und der Proband stratifiziert wird,
f.) die Diagnose oder Prädiktion von Darmerkrankungen durch ein weiteres Verfahren zur Diagnose oder Prädiktion von mindestens einer Darmerkrankung ausgewählt aus der Gruppe Reizdarmsyndrom (IBS), Laktose- oder Fruktosemalabsorption, chronisch entzündliche Darmerkrankungen (CED), insbesondere Morbus Crohn und Colitis ulcerosa, Zöliakie, funktionelle Dyspepsie durchzuführen.

5. Nicht-invasives Verfahren zur Diagnose oder Prädiktion von Darmerkrankungen nach einem der Ansprüche 1 bis 4, wobei eine Zunahme der ppm Gehalte von H₂ und/oder CH₄ innerhalb von 60 bis 100 Minuten erfolgt, insbesondere 80 Minuten.

6. Nicht-invasives Verfahren zur Diagnose oder Prädiktion von Darmerkrankungen nach einem der Ansprüche 1 bis 5, für die Therapiesteuerung, mit der Maßgabe, dass der Proband Medikamente, insbesondere Antibiotika, Phytobiotika zur Behandlung einer Darmerkrankung erhält oder eine Diät zur Behandlung einer Darmerkrankung einhält, insbesondere zur Behandlung einer Malabsorption.

7. Nicht-invasives Verfahren zur Diagnose oder Prädiktion von Darmerkrankungen nach einem der Ansprüche 1 bis 5 zur Risikostratifizierung von Probanden.
